# EUROPEAN PATENT APPLICATION

(11) **EP 3 878 470 A1**
(43) Date of publication of application: **15.09.2021**
(21) Application number: 19882083.9
(22) Date of filing: 07.11.2019
(51) Int. Cl.: A61K 45/00, A61P 9/10, A61P 19/10, A61P 43/00, C12N 5/071

(54) **METHOD FOR REMOVING SENESCENT CELL, AND METHOD FOR PREPARING SENESCENT CELL**

(30) Priority: 08.11.2018 JP 2018210300
(71) Applicant: The University of Tokyo, Bunkyo-ku, Tokyo 113-8654 (JP)
(72) Inventor: NAKANISHI Makoto, Tokyo 113-8654 (JP); JOHMURA Yoshikazu, Tokyo 113-8654 (JP)
(74) Representative: Vossius & Partner Patentanwälte Rechtsanwälte mbB
(86) International application number: PCT/JP2019/043570
(87) International publication number: WO 2020/095971

(57) **Abstract**

Solutions to the problem of the invention are a method for selectively killing or removing a senescent cell, substance identification, and a method for purifying a senescent cell. Specifically, the invention includes an agent for removing a senescent cell, which is a drug for removing an *in vivo* senescent cell, the agent containing an inhibitor for glutaminase as an active ingredient, and a pharmaceutical composition containing the agent. The invention further includes a method for preparing a senescent cell, including the following steps (a) to (c): (a) synchronizing a cell with the G2 phase; (b) activating an intracellular p53 protein in the cell synchronized with the G2 phase; and (c) inhibiting polo-like kinase 1 (PLK1) activity in the cell treated in the step (b).

SEQUENCE LISTING

<110>  The University of Tokyo

<120>  A method for removing a senescent cell and a method for preparing
        a senscent cell

<130>  TPC0299UVT

<150>  JP2018-210300
<151>  2018-11-08

<160>  14

<170>  PatentIn version 3.5

<210>  1
<211>  20
<212>  DNA
<213>  Artificial Sequence

<220>
<223>  Synthetic primer

<400>  1
cccaacgcac cgaatagtta                                                   20


<210>  2
<211>  18
<212>  DNA
<213>  Artificial Sequence

<220>
<223>  Synthetic primer

<400>  2
accagcgtgt ccaggaag                                                     18


<210>  3
<211>  20
<212>  DNA
<213>  Artificial Sequence

<220>
<223>  Synthetic primer

<400>  3
ccaggagccc agctatgaac                                                   20


<210>  4
<211>  18
<212>  DNA
<213>  Artificial Sequence

<220>
<223>  Synthetic primer

<400>  4
cccagggaga aggcactg                                                     18

<210>  5
<211>  20
<212>  DNA
<213>  Artificial Sequence

<220>
<223>  Synthetic primer

<400>  5
aaggaaaact gggtgcagag                                                    20


<210>  6
<211>  20
<212>  DNA
<213>  Artificial Sequence

<220>
<223>  Synthetic primer

<400>  6
attgcatctg gcaaccctac                                                    20


<210>  7
<211>  18
<212>  DNA
<213>  Artificial Sequence

<220>
<223>  Synthetic primer

<400>  7
ccgctgcaga cagactgg                                                      18


<210>  8
<211>  21
<212>  DNA
<213>  Artificial Sequence

<220>
<223>  Synthetic primer

<400>  8
ccatcatcat cacctgaatc g                                                  21


<210>  9
<211>  20
<212>  DNA
<213>  Artificial Sequence

<220>
<223>  Synthetic primer

<400>  9
cgcaggttct tggtcactgt                                                    20


<210>  10
<211>  20

<212>  DNA
<213>  Artificial Sequence

<220>
<223>  Synthetic primer

<400>  10
tgttcacgaa agccagagcg                                                   20


<210>  11
<211>  26
<212>  DNA
<213>  Artificial Sequence

<220>
<223>  Synthetic primer

<400>  11
actggagatg tgtctgccct ccgaag                                            26


<210>  12
<211>  27
<212>  DNA
<213>  Artificial Sequence

<220>
<223>  Synthetic primer

<400>  12
ccaaagtgta gtgcttcatc catgggg                                           27


<210>  13
<211>  20
<212>  DNA
<213>  Artificial Sequence

<220>
<223>  Synthetic primer

<400>  13
ccaagaggtg agtgcttccc                                                   20


<210>  14
<211>  22
<212>  DNA
<213>  Artificial Sequence

<220>
<223>  Synthetic primer

<400>  14
ctgttgttca gactctctcc ct                                                22

## Description

### Technical Field

The present invention relates to a method for removing a senescent cell from an individual. Furthermore, the present invention relates to a method for preparing a purified senescent cell.

### Background Art

For today's super-aged society, extending healthy life expectancy is one of the most important issues to be solved by modern science. It is obvious that reforming the medical system and improving lifestyle-related diseases and eating habits are effective means for extending healthy life expectancy. However, in order to drastically respond to healthy life expectancy, it is essential to have a bird's-eye view of the aging control mechanism and to develop technology to prevent age-related diseases and functional deterioration of organs and tissues.

Until now, cellular senescence has been considered to be an irreversible arrest of cell proliferation and has been positioned as one of the cancer defense mechanisms. Meanwhile, cellular senescence has also come to be considered to play a role in individual-level aging and diseases associated with aging. Senescent cells have been shown to secrete inflammatory cytokines, chemokines, matrix metalloproteinases, and growth factors, etc. This phenomenon is called the senescence-associated secretory phenotype (SASP) and has been suggested to be associated with the development of age-related diseases (Non Patent Literature 1 to 3).

In recent years, a major paradigm shift has occurred in the elucidation of the aging control mechanism. Baker et al. reported that the genetic analysis using progeroid model mice shows that artificial removal of senescent cells from aged animals significantly delays the onset of geriatric diseases such as arteriosclerosis and renal damage, and also prolongs lifespan itself (Non Patent Literature 4). Further, as a result of the examination of effects of senescent cells on healthy life expectancy in nonprogeroid mice, it was suggested that Accumulation of p16-positive cells, one of the biomarkers of senescent cells, tends to shorten their lifespan, and that the removal of p16-positive cells may extend healthy life expectancy for individuals (Non Patent Literature 5). Therefore, the development of drugs that can selectively kill or eliminate senescent cells *in vivo* is thought to lead to the establishment of new methodologies for extending healthy life expectancy and treating age-related diseases (arteriosclerosis and osteoporosis).

Incidentally, since cellular senescence is induced by various genomic stresses, a specific signal pathway is activated depending on the type of induced stimulus. Therefore, in order to clarify the transcriptome and metabolome state common to all senescent cells, it is indispensable to develop a 100% purified senescent cell preparation technique without external stimulus.

So far, the present inventors have revealed that activation of p53 in the G2 phase is necessary and sufficient for the induction of cellular senescence (Non Patent Literature 6). However, at present, no technique for stably preparing purified senescent cells has been established.

### Summary of Invention

### Non Patent Literature

Non Patent Literature 1: Campisi et al., Annu. Rev. Physiol. 75: 685-705, 2013
Non Patent Literature 2: Shapless et al., nature Rev. Cancer 15: 397-408, 2015
Non Patent Literature 3: van Deursen, Nature 509: 439-446, 2014
Non Patent Literature 4: Baker et al., Nature 479: 232-236, 2011
Non Patent Literature 5: Baker et al., Nature 530: 184-189, 2016
Non Patent Literature 6: Johmura et al., Mol Cell 55: 73-84, 2014

### Summary of Invention

### Technical Problem

In view of the above circumstances, the present inventors aim to establish a method for purifying a senescent cell, and at the same time, set solutions to the problem which are a method for selectively killing or removing a senescent cell and substance identification.

### Solution to Problem

The inventors first attempted to purify senescent cells. So far, it has been shown that activating the tumor suppressor protein p53 while the cells are synchronized with the G2 phase is necessary and sufficient for inducing cellular senescence (Non Patent Literature 6). Based on the above knowledge, the present inventors investigated a method for preparing a purified senescent cell population (cell population consisting of senescent cells) after inducing cellular senescence, and thus found that purification of senescent cells is possible by activating p53 of G2 phase cells and further inhibiting the activity of polo-like kinase 1 (PLK1).

Furthermore, the inventors performed metabolome analysis of senescent cells by gas chromatography using the above-described culture system of purified senescent cells. As a result, it was suggested that in senescent cells, the conversion reaction from citric acid to isocitric acid is inhibited by the increase in the amount of active oxygen, and the production of α-ketoglutaric acid and the subsequent rotation of the citric acid cycle may depend on the glutamine metabolic pathway (glutaminolysis). Therefore, it was clarified that inhibition of glutaminolysis of senescent cells with a drug induces selective cell death in senescent cells.

The present invention has been completed based on the above findings.

Specifically, the present invention includes the following (1) to (8).
(1) An agent for removing a senescent cell, which is a drug for removing an *in vivo* senescent cell, the agent containing an inhibitor for glutaminase as an active ingredient.
(2) The agent for removing a senescent cell according to the above (1), wherein the glutaminase is kidney-type glutaminase (KGA).
(3) A pharmaceutical composition for preventing or treating a disease that develops with aging, which contains the agent for removing a senescent cell according to the above (1) or (2).
(4) The pharmaceutical composition according to the above (3), wherein the disease is atherosclerosis, osteoporosis, cataract, glaucoma, dementia, Parkinson's disease, lung fibrosis, chronic obstructive pulmonary disease, cancer, type 2 diabetes, chronic renal failure, cardiomegaly, liver cirrhosis, sarcopenia, or emaciation.
(5) A method for preparing a senescent cell, comprising the following steps (a) to (c):
   (a) synchronizing a cell with the G2 phase;
   (b) activating an intracellular p53 protein in the cell synchronized with the G2 phase; and
   (c) inhibiting polo-like kinase 1 (PLK1) activity in the cell treated in the step (b).
(6) The method according to the above (5), wherein the step (a) is a step of bringing a cyclin-dependent kinase 1 (CDK1) activity inhibitor into contact with the cell.
(7) The method according to the above (5), wherein the step (b) is a step of bringing an Mdm2 protein inhibitor into contact with the cell.
(8) The method according to the above (5), wherein the step (c) is a step of bringing a PLK1 activity inhibitor into contact with the cell.

### Advantageous Effects of Invention

According to the present invention, it is possible to efficiently prepare a purified senescent cell population.

According to the present invention, *in vivo* senescent cells can be removed. As a result, it is expected that the healthy life expectancy of an individual will be extended, and it will be possible to prevent age-related diseases and develop treatment methods and therapeutic agents for the diseases.

### Brief Description of Drawings

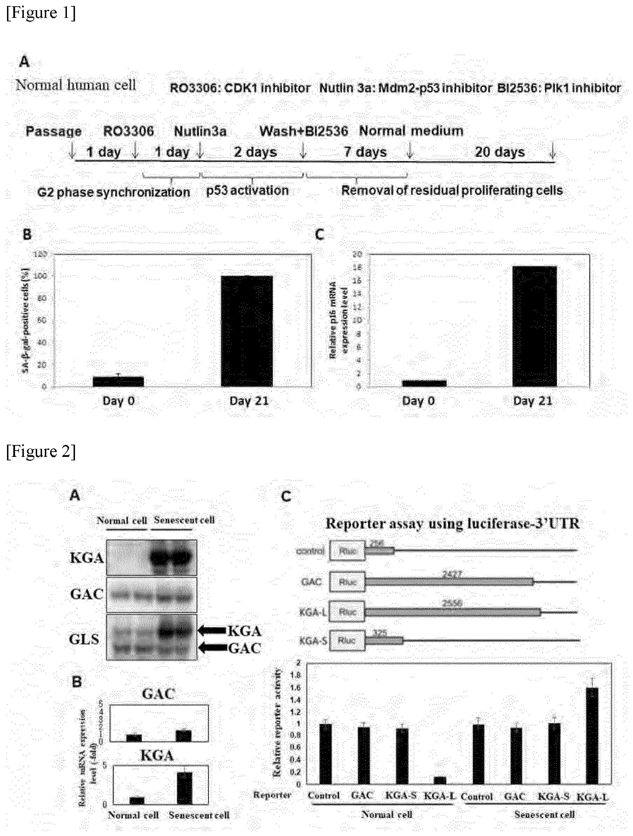

[Figure 1] Figure 1 shows preparation of senescent cells. Figure 1A shows an example of the preparation schedule for 100% purified senescent cells. The proportion of SA-β-gal positive cells (B) and the p16 mRNA expression level in the cells on Day 21 from the start of preparation are also shown.
[Figure 2] Figure 2 shows examination of the glutaminase expression level in senescent cells. Figure 2A shows the results of Western blotting with antibodies against KGA and GAC using cell extracts from cells after senescence induction (senescent cells) and cells before senescence induction (normal cells). Figure 2B shows the results of measuring the mRNA expression levels of KGA and GAC in senescent cells and normal cells by qPCR. Figure 2C shows the results of examining the stability of each glutaminase mRNA in senescent cells and normal cells by luciferase reporter assay.

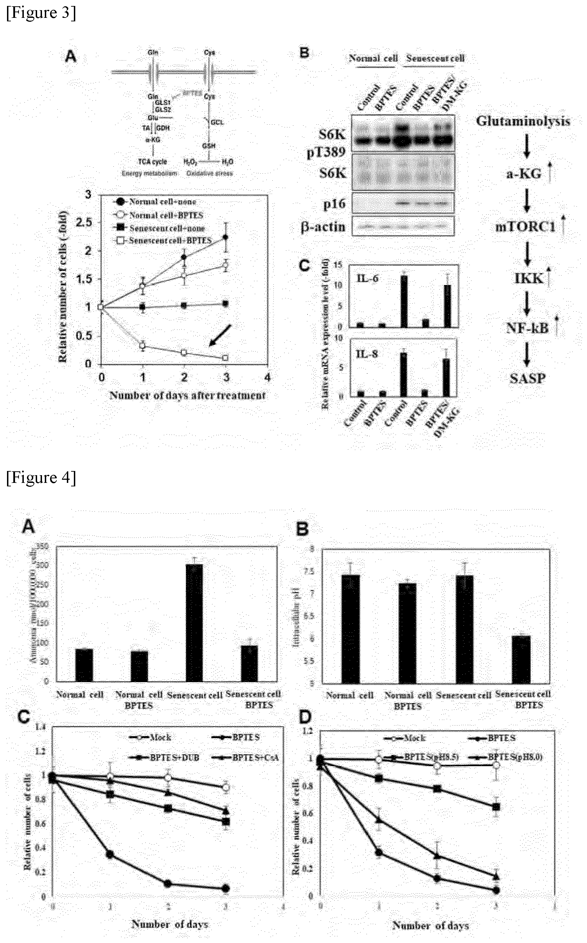

[Figure 3] Figure 3 shows examination of the role of glutaminolysis in senescent cells. Figure 3A shows a diagram schematically showing glutaminolysis (upper panel) and the results of examining the effects of the glutaminase inhibitor (BPTES) on senescent cells and normal cells (lower panel). Figure 3B shows the results of detection of phosphorylation of S6K protein T389, S6K protein, and p16 protein amount in the presence or absence of BPTES or BPTES + DM-KG (transmembrane α-ketoglutarate) with antibodies using cell extracts from senescent cells and normal cells. Figure 3C shows the results of measuring the mRNA expression levels of IL-6 and IL-8 in the presence or absence of BPTES or BPTE + DM-KG (transmembrane α-ketoglutarate) in senescent cells and normal cells.
[Figure 4] Figure 4 shows the role of glutaminolysis in controlling pH in senescent cells. Figure 4A shows the results of measuring the intracellular ammonia concentrations in senescent cells and normal cells in the presence or absence of BPTES. Figure 4B shows the results of measuring intracellular pH of senescent cells and normal cells in the presence or absence of BPTES. Figure 4C shows the results of counting the number of senescent cells in the absence of BPTES, in the presence of BPTES, or in the presence of BPTES + DUB or BPTES + CsA. Figure 4D shows the results of counting the number of senescent cells in the absence of BPTES, in the presence of BPTES, or in the presence of BPTES + DUB or BPTES + CsA.

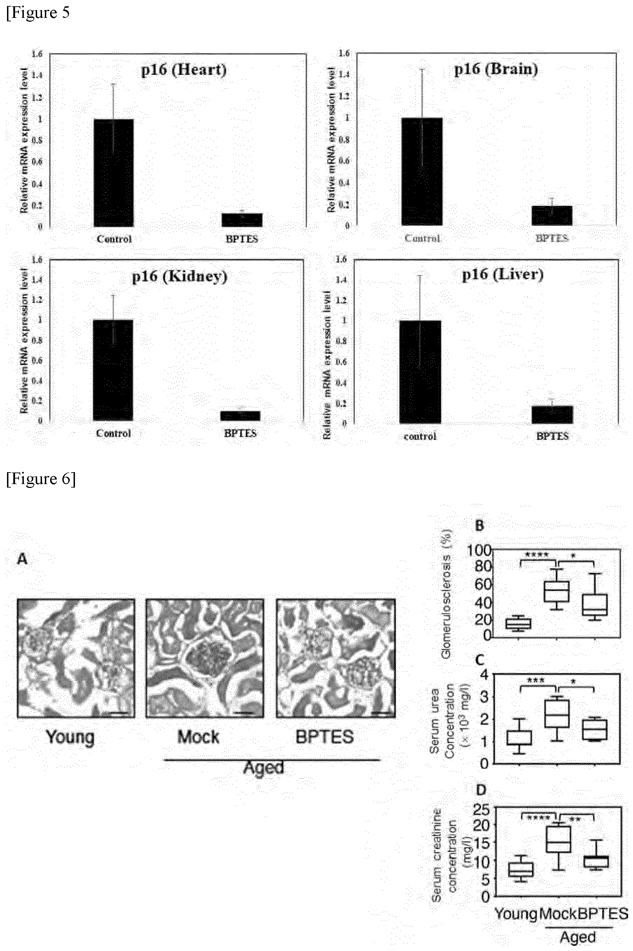

[Figure 5] Figure 5 shows the removal of senescent cells by inhibiting glutaminolysis with a glutaminase inhibitor. The results of measuring the expression level of the p16 gene (senescence marker) in the heart, brain, kidney, and liver of mice administered with BPTES (12.5 mg/kg body weight).
[Figure 6] Figure 6 shows the effects of glutaminolysis inhibitor on age-associated glomerulosclerosis. Figure 6A shows the results of PAS staining of glomeruli collected from young mice (8 weeks old, n = 8), vehicle-treated aged mice (Aged, Mock) (76 weeks old, n = 12), or BPTES-treated aged mice (Aged, BPTES) (76 weeks old, n = 12). The scale bar is 250 µm. Figures 6B, 6C, and 6D show the degree of glomerulosclerosis (B), serum urea concentration (C), and serum creatinine concentration (D), respectively. Data are shown as mean ± standard deviation, and box plots indicate median, interquartile values and range. After the analysis of data by one-way ANOVA, multiple comparisons were performed by the Tukey's multiple comparisons post hoc test. *P < 0.05, ***P < 0.001.

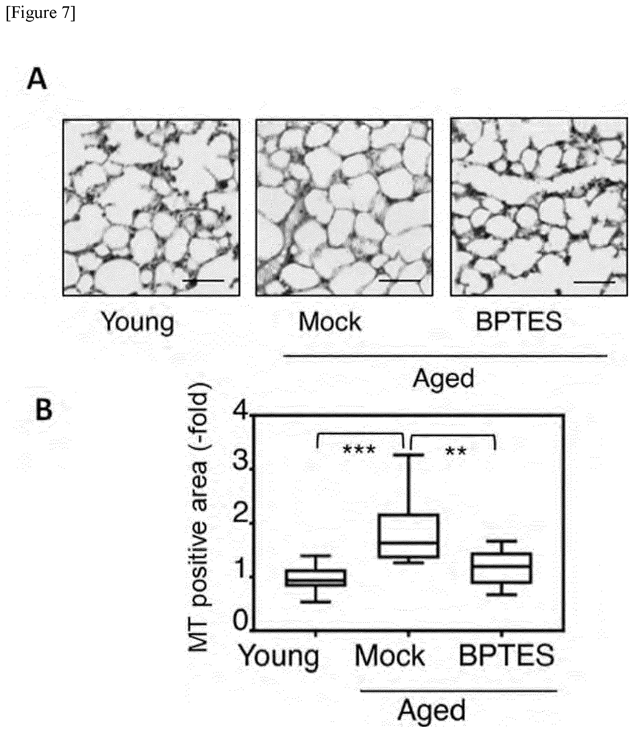

[Figure 7] Figure 7 shows the effects of glutaminolysis inhibitor on age-associated lung fibrosis. Figure 7A shows the results of MT staining of lung tissues collected from young mice (8 weeks old, n = 8), vehicle-treated aged mice (Aged, Mock) (76 weeks old, n = 12), or BPTES-treated aged mice (Aged, BPTES) (76 weeks old, n = 12). The scale bar is 100 µm. Figure 7B indicates the MT-staining positive area. Each value is shown as a relative value with an average Young's value of 1. Data are shown as mean ± standard deviation, and box plots indicate median, interquartile values and range. Statistical processing is the same as in Figure 6. **P < 0.01, ****P < 0.0001.

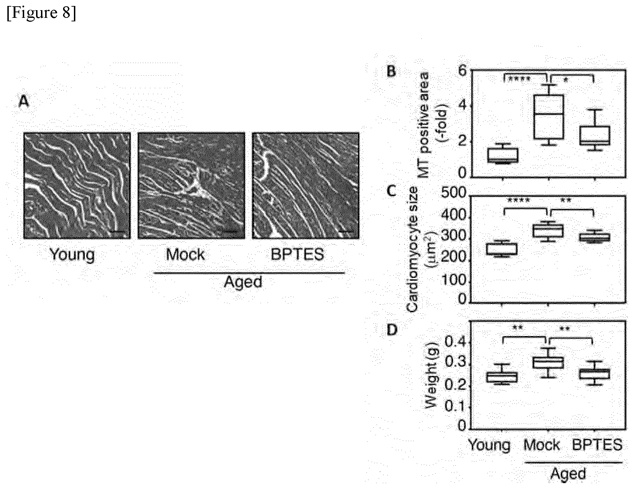

[Figure 8] Figure 8 shows the effects of glutaminolysis inhibitor on age-associated myocardial fibrosis and cardiomegaly. Figure 7A shows the results of MT staining of heart tissues collected from young mice (8 weeks old, n = 8), vehicle-treated aged mice (Aged, Mock) (76 weeks old, n = 12), or BPTES-treated aged mice (Aged, BPTES) (76 weeks old, n = 12). The scale bar is 100 µm. Figures 8B, 8C, and 8D show the MT staining-positive area (B), cardiomyocyte size (C), and heart weight (D), respectively. Data are shown as mean ± standard deviation, and box plots indicate median, interquartile values and range. Each value in Figure 8B is shown as a relative value with an average Young's value of 1. Statistical processing is the same as in Figure 6. *P < 0.05, **P < 0.01, ****P < 0.0001.

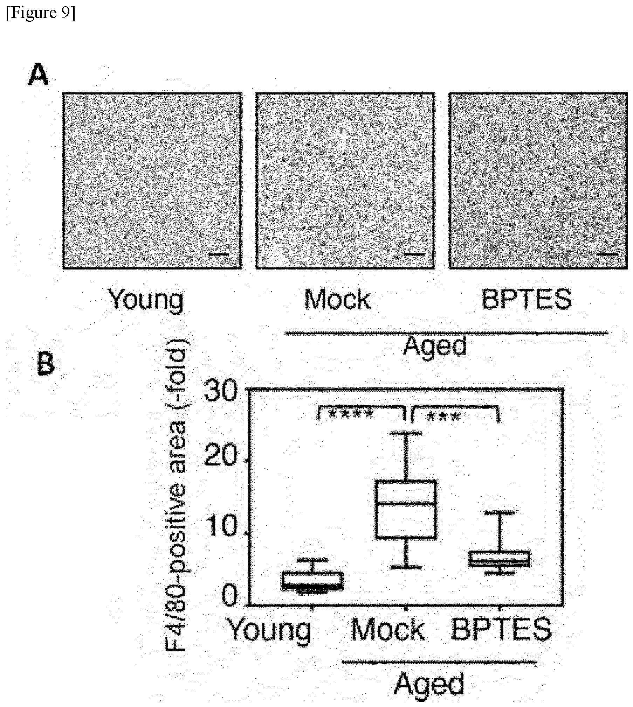

[Figure 9] Figure 9 shows the effects of glutaminolysis inhibitor on macrophage infiltration into the liver. Figure 9A shows the results of immunostaining of liver tissues collected from young mice (8 weeks old, n = 8), vehicle-treated aged mice (Aged, Mock) (76 weeks old, n = 12), or BPTES-treated aged mice (Aged, BPTES) (76 weeks old, n = 12) with the anti-F4/80 antibody. The scale bar is 50 µm. In Figure 9B, the area stained with the anti-F4/80 antibody is shown as a relative value with an average Young's value of 1. Data are shown as mean ± standard deviation, and box plots indicate median, interquartile values and range. Statistical processing is the same as in Figure 6. ***P < 0.001, ****P < 0.0001.

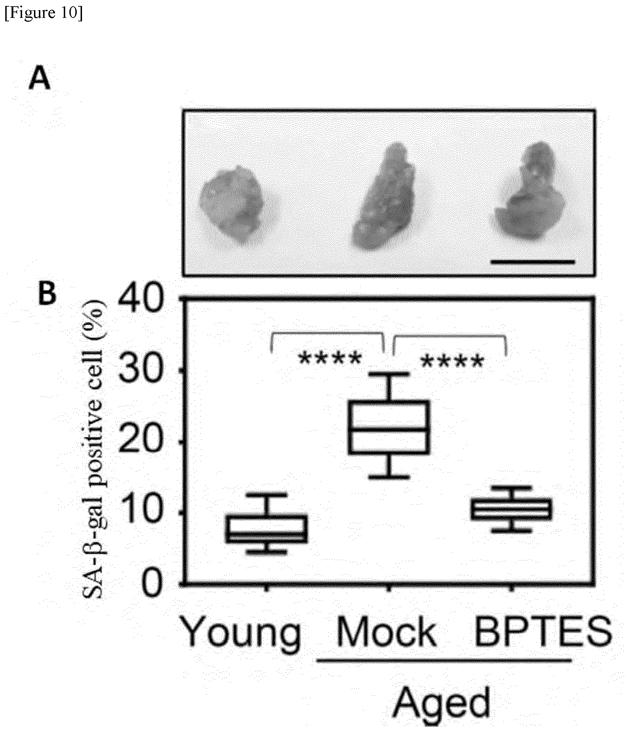

[Figure 10] Figure 10 shows the effects of glutaminolysis inhibitor on age-related accumulation of senescent cells in white adipose tissue. The results of *in situ* SA-β-gal staining of adipose tissues collected from young mice (8 weeks old, n = 8), vehicle-treated aged mice (Aged, Mock) (76 weeks old, n = 12), or BPTES-treated aged mice (Aged, BPTES) (76 weeks old, n = 12) (A), and the proportion of SA-β-gal positive cells (B) are shown. The scale bar is 0.5 cm. Data are shown as mean ± standard deviation, and box plots indicate median, interquartile values and range. Statistical processing is the same as in Figure 6. ****P < 0.0001.

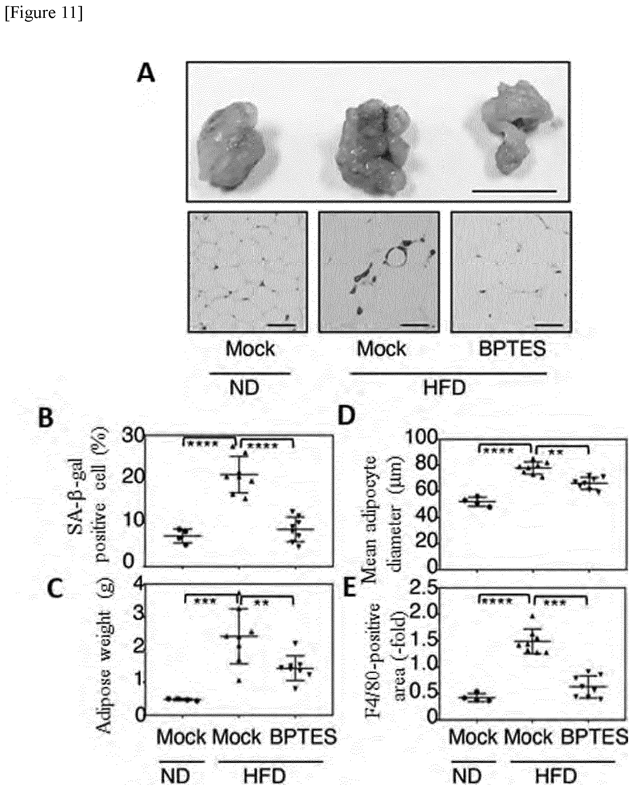

[Figure 11] Figure 11 shows the effects of glutaminolysis inhibitor on obesity-associated accumulation of senescent cells in white adipose tissue and macrophage infiltration and hypertrophy. Figure 11A shows the results of *in situ* SA-β-gal staining of adipose tissues collected from mice (8 weeks old, n = 4) fed a normal diet (ND), vehicle-treated (Mock) mice fed a high fat diet (HFD) (8 weeks old, n = 8), or BPTES-treated (BPTES) mice fed a high fat diet (HFD) (8 weeks old, n = 8) (upper panel) and the results of immunostaining with the anti-F4/80 antibody (lower panels). Figures 11B, 11C, 11D, and 11E show the SA-β-gal positive area (B), weight of adipose tissue (C), mean adipocyte diameter (D), and area stained with the anti-F4/80 antibody (E), respectively. Data are shown as mean ± standard deviation, and box plots indicate median, interquartile values and range. Statistical processing is the same as in Figure 6. **P < 0.01, ***P < 0.001, ****P < 0.0001.

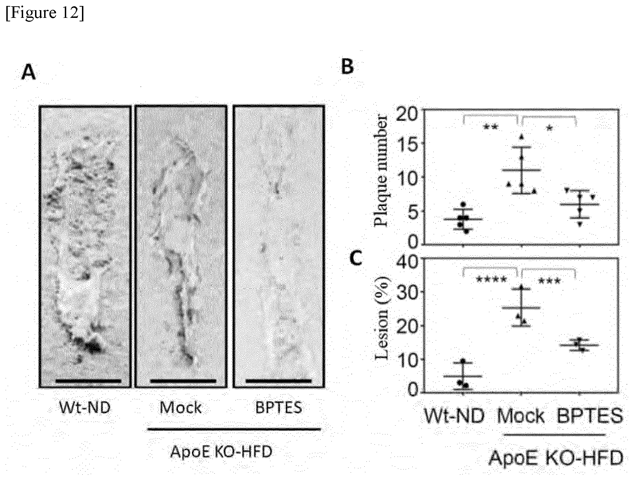

[Figure 12] Figure 12 shows the effects of glutaminolysis inhibitor on obesity-associated atherosclerosis. Figure 12A shows the results of Sudan IV staining of aortas of wild-type mice (Wt-ND) (8 weeks old, n = 5), vehicle-treated (Mock) ApoE knockout mice fed a high fat diet (8 weeks old, n = 5), or BPTES-treated (BPTES) ApoE knockout mice fed a high fat diet (8 weeks old, n = 5). Figure 12B and 12C show the aortic plaque number (B) and the proportion of lesion (C), respectively. The scale bar is 500 µm. Data are shown as mean ± standard deviation, and box plots indicate median, interquartile values and range. Statistical processing is the same as in Figure 6. *P < 0.05, **P < 0.01, ***P < 0.001, ****P < 0.0001.

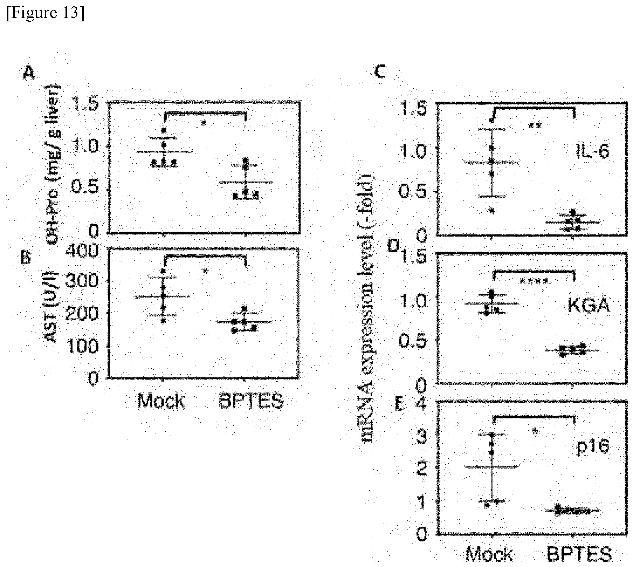

[Figure 13] Figure 13 shows the effects of glutaminolysis inhibitor on liver dysfunction associated with non-alcoholic steatohepatitis. The results of measuring the levels of hydroxyproline (OH-Pro) (A) and serum AST (B) in the liver, and the mRNA expression levels of IL-6 (C), KGA (D), and p16 (E) in the liver after feeding wild-type mice (8 weeks old) a choline-deficient L-amino acid-defined high-fat diet for 8 weeks and treating them with vehicles (Mock) (n = 5) or BPTES (BPTES) (n = 5) are shown. Data are shown as mean ± standard deviation, and box plots indicate median, interquartile values and range. Statistical processing is the same as in Figure 6. *P < 0.05, **P < 0.01, ****P < 0.0001.

### Description of Embodiments

A first embodiment of the present invention relates to an agent for removing a senescent cell, which is a drug for removing an *in vivo* senescent cell, the agent containing an inhibitor of glutaminolysis (glutamine metabolic pathway), for example, an inhibitor for glutaminase as an active ingredient (hereinafter also referred to as the "agent for removing a senescent cell of the present invention").

The "agent for removing a senescent cell" described herein means a drug which induces cell death in a senescent cell *in vivo* or *in vitro* and selectively removes the senescent cell from a cell population containing the senescent cell.

According to the embodiments of the present invention, the term "senescent cell" refers to a cell with irreversible cell proliferation or cell cycle arrest. It is possible to evaluate whether or not a cell is a senescent cell by using the characteristics of cellular senescence as an indicator. Many previous studies have reported the characteristics of cellular senescence including, for example, increased p16 (CDKN2A) protein expression, activation of senescence-associated β-galactosidase (SA-β-gal), increased p21 (CDKN1A) protein expression, increased p19 protein expression, formation of senescence-associated heterochromatic foci (SAHF), DNA damage response (DDR), and senescence-associated secretory phenotype (SASP) (regarding the characteristics of cellular senescence, see, for example, Kuilman et al., Genes Dev 24:2463-2479, 2010 for details).

The present inventors clarified that inhibition of glutaminolysis in senescent cells induces selective cell death in senescent cells as stated above. Glutaminolysis is composed of several reaction stages, and in particular, senescent cell-specific cell death can be efficiently induced by inhibiting the reaction stage of producing glutamate from glutamine. The reaction to produce glutamic acid from glutamine is catalyzed by glutaminase (EC 13.5.1.2). There are two types of mammals, the kidney-type glutaminase (KGA) encoded by the GLS1 gene and the liver-type glutaminase (LGA) encoded by the GLS2 gene. KGA is widely distributed throughout the body, whereas LGA is mainly present in the liver. In addition, KGA exists as two splice variants that differ only in the C-terminal region, and the long form is called KGA as it is, and the short form is called GAC (glutaminase C).

The glutaminase inhibitor used in the embodiments of the present invention may be any one as long as it inhibits the activity of at least KGA, and such an inhibitor can be easily selected by those skilled in the art. Examples of the glutaminase inhibitor include, but are not particularly limited to, BPTES (bis-2-(5-phenylacetamido-1,3,4-thiadiazol-2-yl)ethyl sulfide) (CAS No: 314045-39-1), DON (6-diazo-5-oxo-L-norleucine) (CAS No: 51481-10-8), compound 968 (CAS No: 311795-38-7), and CB-839 (CAS No: 1439399-58-2).

In addition to these, proteins, or peptides such as neutralizing antibodies against KGA or fragments thereof, and nucleic acids such as siRNA and miRNA for knocking out the gene encoding KGA (GLS1) may be used as glutaminase inhibitors.

A second embodiment of the present invention relates to a pharmaceutical composition containing the agent for removing a senescent cell of the present invention (hereinafter also referred to as the "pharmaceutical composition of the present invention"). Since the pharmaceutical composition of the present invention contains, as an active ingredient, the agent for removing a senescent cell of the present invention, when it is administered *in vivo, in vivo* senescent cells are selectively killed or removed (see the Examples). Therefore, the pharmaceutical composition of the present invention is expected to be effective for preventing or treating diseases that develop with extended healthy life expectancy and aging such as atherosclerosis, osteoporosis, cataract, glaucoma, dementia, Parkinson's disease, lung fibrosis, chronic obstructive pulmonary disease, cancer, type 2 diabetes, chronic renal failure, cardiomegaly, liver cirrhosis, sarcopenia, and emaciation. It should be noted that the diseases listed herein are merely examples, and it goes without saying that diseases other than these may also be the subject of the present invention as aging or age-related diseases caused by the accumulation of senescent cells.

The pharmaceutical composition of the present invention may be administered in the form of a pharmaceutical composition comprising one or more pharmaceutical additives in addition to the active ingredient (agent for removing a senescent cell). Other known agents may be added to the pharmaceutical composition according to the embodiments.

The pharmaceutical composition of the present invention may be in an oral or parenteral dosage form and is not particularly limited. Examples thereof include tablets, capsules, granules, powders, syrups, suspensions, suppositories, ointments, creams, gels, patches, inhalants, and injections. These formulations are prepared according to a conventional method. In the case of liquid formulations, they may be dissolved or suspended in water or other suitable solvents at the time of use. In addition, tablets and granules may be coated by a well-known method. In the case of injections, the active ingredient is prepared by dissolving it in water, but if necessary, it may be dissolved in physiological saline or a glucose solution, or a buffer or a preservative may be added.

Type of a pharmaceutical additive used for producing the pharmaceutical composition of the present invention, the ratio of the pharmaceutical additive to the active ingredient, or the method for producing the pharmaceutical composition shall be appropriately selected by those skilled in the art according to the dosage form. Inorganic or organic substances, or solid or liquid substances can be used as pharmaceutical additives, and generally, for example, they can be blended at 0.1% by weight to 99.9% by weight, 1% by weight to 95.0% by weight, or 1% by weight and 90.0% by weight with respect to the weight of the active ingredient. Specific examples of pharmaceutical additives include lactose, glucose, mannitol, dextrin, cyclodextrin, starch, sucrose, magnesium aluminometasilicate, synthetic aluminum silicate, sodium carboxymethyl cellulose, hydroxypropyl starch, carboxymethyl cellulose calcium, ion exchange resin, methyl cellulose, gelatin, gum arabic, hydroxypropyl cellulose, hydroxypropyl methylcellulose, polyvinylpyrrolidone, polyvinyl alcohol, light silicic acid anhydride, magnesium stearate, talc, tragant, bentonite, VEEGUM, titanium oxide, sorbitan fatty acid ester, sodium lauryl sulfate, glycerin, fatty acid glycerin ester, purified lanolin, glycerol gelatin, polysorbate, macrogol, vegetable oil, wax, liquid paraffin, white petrolatum, fluorocarbon, nonionic surfactant, propylene glycol, and water.

When producing a solid preparation for oral administration, the active ingredient, and an excipient ingredient, for example, lactose, starch, crystalline cellulose, calcium lactate, or silicic acid anhydride are mixed to form a powder, and further, if necessary, a binder such as sucrose, hydroxypropyl cellulose, or polyvinylpyrrolidone and a disintegrant such as carboxymethyl cellulose or carboxymethyl cellulose calcium are added, and wet or dry granulation is performed to obtain granules. To produce tablets, these powders and granules may be used as they are, or they may be tableted by adding a lubricant such as magnesium stearate or talc. These granules or tablets may be coated with an enteric solvent base such as hydroxypropylmethylcellulose phthalate or methacrylic acid-methylmethacrylic acid polymer to form an enteric solvent preparation, or they may be coated with ethyl cellulose, carnauba wax, or a curing oil to form a longacting preparation. In addition, to produce capsules, hard capsules can be filled with powders or granules, or the active ingredient can be used as is, or dissolved in glycerin, polyethylene glycol, sesame oil, olive oil, or the like, and then coated with gelatin, thereby preparing soft capsules.

To produce an injection, the active ingredient can be dissolved in distilled water for injection with a pH regulator such as hydrochloric acid, sodium hydroxide, lactose, lactic acid, sodium, sodium monohydrogen phosphate, or sodium dihydrogen phosphate and an isotonic agent such as sodium chloride or glucose, aseptically filtered, and filled into ampoules as necessary, and further, mannitol, dextrin, cyclodextrin, gelatin, or the like can be added, followed by freeze-drying in vacuum, thereby preparing an injection dissolved before use. It is also possible to add reticine, polysorbate 80, polyoxyethylene hydrogenated castor oil, or the like to the active ingredient and emulsify it in water to obtain an emulsion for injection.

To produce a rectal agent, the active ingredient can be humidified and dissolved with a suppository base such as cocoa butter, fatty acid tri-, di- or mono-glyceride, or polyethylene glycol, poured into a mold, and cooled, or the active ingredient can be dissolved in polyethylene glycol or soybean oil and then coated with a gelatin film or the like.

The dosage and frequency of administration of the pharmaceutical composition of the present invention are not particularly limited, and may be appropriately selected at the discretion of the physician or pharmacist according to conditions such as prevention of exacerbation/progression of the disease to be treated and/or purpose of treatment, type of disease, weight, and age of the patient.

In general, the daily dose for adults in oral administration is about 0.01 to 1,000 mg (weight of active ingredient), and can be administered once or divided into several times a day, or every few days. In the case of using the pharmaceutical composition of the present invention as an injection, it is desirable to administer a daily dose of 0.001 to 100 mg (weight of active ingredient) continuously or intermittently to adults.

A third embodiment of the present invention relates to a method for inducing cell death in a senescent cell, comprising inhibiting glutaminase activity in the senescent cell *in vitro* or *in vivo.*

Inhibition of glutaminase activity in a senescent cell can also be carried out, for example, by bringing the above-described glutaminase inhibitor into contact with a senescent cell so as to infiltrate the cell. For example, when inducing cell death in an *in vivo* senescent cell, a glutaminase inhibitor may be administered *in vivo* together with a pharmaceutically acceptable carrier or the like. In this case, the glutaminase inhibitor can also be administered in the form of the pharmaceutical composition described in the second embodiment above.

A fourth embodiment of the present invention relates to a method for preventing or treating (prevention or treatment of) a disease that develops with aging, comprising administering the pharmaceutical composition of the present invention or the agent for removing a senescent cell of the present invention to a patient.

Here, "treating" means a treatment for the purpose of stopping or alleviating the progression and exacerbation of a disease in a patient who has already developed a disease that develops with aging, thereby stopping or alleviating the progression and exacerbation of the disease.

In addition, "preventing" means a treatment for the purpose of preventing the onset of a disease that develops with aging in advance, thereby preventing the onset of the disease in advance.

The target of treatment and prevention is not limited to humans, and may be mammals other than humans, such as mice, rats, dogs, cats, as well as domestic animals such as cows, horses, and sheep, and primates such as monkeys, chimpanzees, and gorillas. Humans are particularly preferable.

A fifth embodiment of the present invention relates to a method for preparing a senescent cell, comprising the following steps (a) to (c):
(a) synchronizing a cell with the G2 phase;
(b) activating an intracellular p53 protein in the cell synchronized with the G2 phase; and
(c) inhibiting polo-like kinase 1 (PLK1) activity in the cell treated in the step (b).

It is possible to evaluate whether or not the cell is a senescent cell using, as an indicator, for example, a significant increase in the intracellular expression level of p16 protein, p21 protein, or p19 protein compared to a normal cell, a significant increase in the activity of senescence-associated β-galactosidase (SA-β-gal) compared to a normal cell, a significant increase in secretion of SASP-specific molecules such as inflammatory cytokines (e.g., IL-6 and IL-8), growth factors (e.g., IGFBP7), and matrix metalloproteinases (MMPs), or the like.

Cells in which senescence is induced may be from any animal as long as they are from mammals, and may be from any tissue. The basic medium for cell culture for preparing senescent cells may be any medium as long as it is suitable for the cells to be cultured, and if necessary, antibiotics, protease inhibitors, and the like may be added for use. Further, as for the culture conditions, the CO₂ concentration, and the culture temperature suitable for the cells to be used can be adopted.

The step (a) in the fifth embodiment is a step of carrying out a treatment for synchronizing the cell cycle of a cell population in which senescence is induced with the G2 (gap2) phase. Those skilled in the art can easily select an appropriate method for synchronizing a cell to the G2 phase. Examples thereof can include a method for treating a cell with a cyclin-dependent kinase 1 (CDK1) inhibitor (for example, bringing a CDK1 inhibitor into contact with a cell) so as to inhibit CDK1 activity in the cell as well as addition of an anti-cancer drug, radiation irradiation, and UV irradiation. Commercially available CDK inhibitors may be used to inhibit CDK1 activity. Examples of CDK1 inhibitors can include RO3306 (CAS No: 872573-93-8), Roscovitine (CAS No: 186692-46-6), and BMI-1026 (CAS No: 477726-77-5). It is possible to use a CDK1 inhibitor by adding it to a cell culture medium or the like. The concentration of the CDK1 inhibitor to be used can be easily determined by conducting a preliminary experiment with reference to the instruction manual of the supplier. For example, in the case of using RO3306 as a CDK1 inhibitor, the concentration of RO3306 in a culture medium is not particularly limited, but, for example, 1 to 20 µM, preferably 5 to 10 µM, and more preferably about 9 µM. In the case of using RO3306, the time for treating the cell is not particularly limited, but is, for example, 10 hours to 30 hours, preferably 15 hours to 25 hours, and more preferably about 24 hours.

The step (b) in the fifth embodiment is a step of carrying out a treatment for activating an intracellular p53 protein in the cell (in a cell population) synchronized with the G2 phase. Those skilled in the art can easily select the method of activating the intracellular p53 protein. Examples thereof can include a method for inhibiting activity of Mdm2 protein (that interacts with p53 protein and suppressively regulates p53 protein activity) as well as addition of an anti-cancer drug, radiation irradiation, UV irradiation, oxidative stress loading, and nutrient depletion. Commercially available inhibitors may be used to inhibit Mdm2 protein activity. Examples of such inhibitors can include Nutlin-3a (CAS No: 675576-98-4), HLI373 (CAS No: 502137-98-6), RG7388 (CAS No: 1229705-06-9), AMG-232 (CAS No: 1352066-68-2), and (MI-773 CAS No: 1303607-07-9). It is possible to use a Mdm2 inhibitor by adding it to a cell culture medium or the like. The concentration of the CDK1 inhibitor to be used can be easily determined by conducting a preliminary experiment with reference to the instruction manual of the supplier. For example, in the case of using Nutlin-3a as an Mdm2 inhibitor, the concentration of Nutlin-3a in a culture medium is not particularly limited, but, for example, 1 to 20 µM, preferably 5 to 15 µM, and more preferably about 10 µM. In the case of using Nutlin-3a, the time for treating the cell is not particularly limited, but is, for example, 10 hours to 70 hours, preferably 30 hours to 60 hours, and more preferably about 50 hours.

The step (c) in the fifth embodiment is a step of carrying out a treatment for inhibiting polo-like kinase 1 (PLK1) activity of the cell in which p53 protein is activated in the G2 phase in the steps (a) and (b). Commercially available PLK1 activity inhibitors may be used to inhibit PLK1 activity in the cell. Examples of such inhibitors can include BI2536 (CAS No: 755038-02-9), GSK461364 (CAS No: 929095-18-1), PCM-075 (CAS No: 1263293-37-3), and BI-6727 (CAS: 755038-65-4). It is possible to use a PLK1 activity inhibitor by adding it to a cell culture medium or the like. The concentration of the CDK1 inhibitor to be used can be easily determined by conducting a preliminary experiment with reference to the instruction manual of the supplier. For example, in the case of using BI2536 as a PLK1 activity inhibitor, the concentration of BI2536 in a culture medium is not particularly limited, but, for example, 50 to 120 nM, preferably 75 to 120 nM, and more preferably about 100 nM. In the case of using BI2536, the time for treating the cell is not particularly limited, but is, for example, 7 to 15 days, preferably 8 to 12 days, and more preferably about 9 days.

When an English translation of the present description includes singular terms with the articles "a," "an," and "the," these terms include not only single items but also multiple items, unless otherwise clearly specified from the context.

Hereinafter, the present invention will be further described in the following examples. However, these examples are only illustrative examples of the embodiments of the present invention, and thus, are not intended to limit the scope of the present invention.

### Examples

### 1. Method for Preparing Senescent Cell

Since cell senescence induction is induced by various genomic stresses, specific signaling pathways are activated depending on the type of induction stimulus. Therefore, in order to clarify the transcriptome and metabolome state common to all senescent cells, it is indispensable to develop a 100% purified senescent cell preparation technique without external stimulus. A culture system capable of stably culturing 100% purified senescent cells for a long period of time without external stimulation will be described in detail below.

RO3306 (SIGMA -ALDRICH) (final concentration: 9 µM) was added to normal human fibroblasts (HCA2), followed by culture at 37°C and 5% CO₂ for 16 hours. Next, culture was performed at 37°C and 5% CO₂ for 8 hours in a culture medium containing RO3306 (final concentration: 9 µM) and Nutlin-3a (SIGMA -ALDRICH) (final concentration: 10 µM), and then culture was performed at 37°C and 5% CO₂ for 48 hours in a culture medium containing Nutlin-3a (final concentration: 10 µM). Culture was performed at 37°C and 5% CO₂ for 9 days while replacing the medium with a medium containing BI2536 (SIGMA -ALDRICH) (final concentration: 100 nM) every 3 days. Thereafter, senescence induction was carried out by performing culture at 37°C and 5% CO₂ for 9 days while replacing the medium with a normal medium (Figure 1A).

Cells before senescence induction (Day 0 of culture) and cells after senescence induction (Day 21 of culture) were stained using the Senescence β-Galactosidase Staining kit (CST) according to the attached protocol.

Stained or unstained cells were counted from 200 randomly selected cells for each plate in which the cells were cultured. As a result, on Day 21 of culture, staining of SA-β-gal was observed in almost all the counted cells (Figure 1B).

In addition, total RNA was prepared from the cells on Day 21 of culture which were treated in the same manner using the RNeasy mini kit (Qiagen) according to the attached protocol. Using the prepared total RNA as a template, reverse transcription into cDNA was performed using the ReverTra Ace qPCR RT kit (Takara) according to the attached protocol. Next, using cDNA as a template, qPCR analysis was performed so as to measure the p16 mRNA expression level using Power SYBR Green PCR Master Mix (Applied Biosystems). Primers for detecting the p16 mRNA expression level are shown below.
Forward: 5'-CCCAACGCACCGAATAGTTA-3' (SEQ ID NO: 1)
Reverse: 5'- ACCAGCGTGTCCAGGAAG-3' (SEQ ID NO: 2)

The mRNA expression level was corrected by the amount of GAPDH mRNA. As a result, the expression of p16 was remarkably increased in the cells on Day 21 of culture.

From the above results, it was confirmed that senescence of almost all cells can be induced by the method for preparing a senescent cell of the present invention.

### 2. Induction of cell death specific to senescent cells

Transcriptome analysis of senescent cells by RNA-sequencing was performed using the senescent cells prepared by the method in 1 above. As a result, it was suggested that the expression of metabolism-related genes was significantly changed in senescent cells. Furthermore, in order to clarify the metabolic characteristics of senescent cells, metabolome analysis using GC-MS was performed. As a result, it was found that senescent cells have the following characteristics unlike normal cells.
(i) Accumulation of citric acid is significant.
(ii) The amount of isocitric acid is significantly reduced.
(iii) The amount of each metabolite in the citric acid cycle after α-ketoglutaric acid is almost unchanged.

Consistent with the above results, it was revealed that the activity of aconitase, which is responsible for the conversion reaction from citric acid to isocitric acid, is significantly reduced in senescent cells. It is known that the activity of aconitase is inhibited when aconitase is oxidized by active oxygen. In fact, it has been found that the amount of active oxygen is remarkably increased in senescent cells, and that administration of an inhibitor of active oxygen to senescent cells restores aconitase activity to a considerable extent.

These results suggest that in senescent cells, the conversion reaction from citric acid to isocitric acid is inhibited by the increase in the amount of active oxygen, and the production of α-ketoglutaric acid and the subsequent rotation of the citric acid cycle may depend on the glutamine metabolic pathway (glutaminolysis). Therefore, the effects of glutaminolysis inhibitors on the survival and functional control of senescent cells were analyzed.

### 2-1. Glutaminase in senescent cells

Cell extracts were prepared from cells before senescence induction (normal cells) and cells after senescence induction (senescent cells) using Laemmli-buffer (2% SDS, 10% glycerol, 5% 2-mercaptoethanol, 0.002% bromophenol blue, and 62.5 mM Tris HCl at pH 6.8). Cell extracts (20 to 50 µg) were separated by SDS-PAGE, and after transcription to PVDF membrane, Western blotting was performed using an anti-KGA antibody, an anti-GAC antibody, and an anti-GLS antibody (each obtained from Proteintech), and detection was performed by ECL. As a result, it was clarified that the expression level of KGA, which is an isoform of glutaminase responsible for the conversion reaction from glutamine to glutamic acid, is remarkably increased in senescent cells (Figure 2A).

Next, using total RNA prepared from normal cells and senescent cells as a template, reverse transcription into cDNA was performed using the ReverTra Ace qPCR RT kit, and then the mRNA expression levels of KGA and GAC were analyzed by qPCR using the Power SYBR Green PCR Master Mix. Each mRNA level was corrected by the expression level of GAPDH mRNA. As a result, it was clarified that the expression level of KGA mRNA was increased in senescent cells (Figure 2B).

In addition, reporter assay was performed in which the 3'UTR of the glutaminase gene was ligated downstream of the luciferase gene in order to elucidate the mechanism of increased expression of glutaminase in senescent cells.

The plasmids of a control in which a random sequence was inserted downstream of the Renilla luciferase gene, GAC in which 2427 bp of 3'UTR of GAC gene was inserted, KGA-L in which 2556 bp of 3'UTR of KGA gene was inserted, and KGA-S in which 325 bp of 3'UTR of KGA gene was inserted were separately introduced into senescent cells and normal cells using a 4D-Nulecofector (Lonza). Reporter activity was measured using the Dual-Glo Luciferase Assay System (Promega) using the cells 48 hours after introduction according to the attached protocol. As a result, it was found that the activity of the reporter gene with the long 3'UTR (KGA-L) of the KGA gene, which is known to be involved in the post-translational regulation of mRNA, was decreased in normal cells than in other reporters, while it was rather increased in senescent cell. The results revealed that the stability of KGA mRNA via the 3'UTR region of the senescent cell glutaminase gene was increased (Figure 2C).

### 2-2. Examination of the role of glutaminolysis in senescent cells

In order to investigate the possibility that senescent cell survival depends on glutaminolysis, the effects of glutaminase inhibitors were examined.

Senescent cells and normal cells were seeded in 6-cm culture dishes such that approximately 10,000 cells were in each dish. On the following day (Day 0), the medium was replaced with a medium containing the glutaminase inhibitor BPTES (final concentration: 10 µM) or a normal medium, the cells were stained with trypan blue every 24 hours, and the number of viable cells was counted using a hemocytometer. As a result, administration of BPTES for 3 days showed an increase in the number of cells in normal cells, while a decrease in the number of cells by 90% or more in senescent cells. Therefore, it was revealed that the glutaminase inhibitor BPTES can selectively induce cell death in senescent cells (Figure 3A).

Next, the effects of BPTES with respect to the expression levels of IL-6 and IL-8, the major factors of a phenotype (SASP) that is one of the most important traits of senescent cells in which large amounts of inflammatory cytokines and extracellular matrix degrading enzymes are secreted. Senescent cells and normal cells were cultured at 37°C and 5%CO₂ for 24 hours in a medium containing BPTES (final concentration: 2.5 µM) or a normal medium, and total RNA was prepared from each cell using ISOGEN II (Wako). After reverse transcription from total RNA into cDNA using the SuperScript II cDNA synthesis kit (Invitrogen) was performed, qPCR analysis was performed using the Power SYBR Green PCR Master Mix (Applied Biosystems) so as to measure the IL-6 and IL-8 expression levels. Primers for detecting the IL-6 and IL-8 mRNA expression levels are shown below.

### IL-6

Forward: 5'-CCAGGAGCCCAGCTATGAAC-3' (SEQ ID NO: 3)
Reverse: 5'-CCCAGGGAGAAGGCACTG-3' (SEQ ID NO: 4)

### IL-8

Forward: 5'-AAGGAAAACTGGGTGCAGAG-3' (SEQ ID NO: 5)
Reverse: 5'-ATTGCATCTGGCAACCCTAC-3' (SEQ ID NO: 6)

It was found that administration of BPTES at a final concentration of 2.5 µM, which has little impact on the survival of senescent cells, inhibits mRNA expression of the major factors of SASP, IL-6 and IL-8 (Figure 3C).

Further, the molecular mechanism of SASP inhibition was analyzed. Senescent cells and normal cells were cultured at 37°C and 5% CO CO₂ for 24 hours in a medium containing BPTES (final concentration: 2.5 µM) or a normal medium. Cell extracts were prepared from the cultured cells using Laemmli-buffer (2% SDS, 10% glycerol, 5% 2-mercaptoethanol, 0.002% bromophenol blue, and 62.5 mM Tris HCl at pH 6.8). Cell extracts (20 to 50 µg) were separated by SDS-PAGE, and after transcription to PVDF membrane, Western blotting was performed using an antibody to the T389 phosphorylation site of the S67K protein (CST), an antibody to the S6K protein (CST), and an antibody to the p16 protein (abcam), and detection was performed by ECL. As a result, it was found that BPTES treatment inhibits phosphorylation of the S6K protein T389, which is an indicator of activation of mTOR that is a major regulatory factor of SASP (Figure 3B, S6KpT389, see the BPTES lane for senescent cells). It was also revealed that this inhibitory effect was rescued by administration of transmembrane α-ketoglutaric acid (DM-KG) (Figure 3B, S6KpT389, see the DM-KG lane for senescent cells).

In addition to the examination of inhibitors, suppression of glutaminase expression using the RNAi method also confirmed selective cell death and suppression of SASP in similar senescent cells.

The above results revealed that activation of glutaminolysis is essential for survival and functional expression of senescent cells.

### 2-3. The role of glutaminolysis in controlling pH in senescent cells

As BPTES (10 µM) treatment could hardly rescue senescent cell-selective cell death by administration of transmembrane α-ketoglutaric acid, it was considered that other metabolites by glutaminolysis may be important. It was considered that acidosis is deeply involved in the stabilization of KGA mRNA, and thus glutaminase, including KGA, may control intracellular pH homeostasis by producing ammonia during the conversion of glutamine to glutamic acid. Therefore, the production of ammonia and the intracellular pH were analyzed.

Senescent cells and normal cells were separately seeded in 10-cm culture dishes such that approximately 50,000 cells were in each dish. On the following day, the medium was replaced with a medium containing BPTES (final concentration: 10 µM) or a normal medium, and culture was performed at 37°C and 5% CO₂ for 24 hours. Thereafter, the amount of ammonia in cells was quantified using the ammonia assay kit (abcam). As a result, it was found that the ammonia production increases about 4-fold in senescent cells compared to normal cells, and that BPTES treatment suppresses the increased ammonia production in senescent cells to the same level as in normal cells (Figure 4A).

Next, senescent cells and normal cells were separately seeded in 6-cm well plates such that approximately 50,000 cells were in each plate. On the following day, the medium was replaced with a medium containing BPTES (final concentration: 10 µM) or a normal medium, and culture was performed at 37°C and 5% CO₂ for 24 hours. The medium was removed, culture was performed at 37°C and 5% CO₂ for 10 minutes in a HEPES solution containing DCECF-AM (DOJINDO LABORATORIES) at a final concentration of 3 µM, and washing with the HEPES solution was performed three times. Thereafter, luminescence was measured with a plate reader. In order to determine the pH value, the amount of luminescence of cells treated with the HEPES solution (pH 6.0 to 7.6) containing nigericin at a final concentration of 10 µM was corrected. As a result, it was also clarified that BPTES treatment reduces intracellular pH in senescent cells from the normal level to from about 7.4 to about 6.0 (Figure 4B).

The previous reports have shown that lowering intracellular pH causes apoptosisindependent cell death through the opening of mitochondrial permeability transition pore (mPTP) by the BNIP3 protein. Therefore, the effects of mPTP inhibitors (DUB and CsA) on BPTES-treated cells were examined.

Senescent cells and normal cells were separately seeded in 6-cm culture dishes such that approximately 10,000 cells were in each dish. On the following day, the medium was replaced with a medium containing BPTES (final concentration: 10 µM), a medium containing BPTES (final concentration: 10 µM) and DUB (final concentration: 10 µM), a medium containing BPTES (final concentration: 10 µM) and CsA (final concentration: 10 µM), or a normal medium, and culture was performed at 37°C and 5% CO₂ for 24 hours. Thereafter, the cells were stained with trypan blue every 24 hours, and the number of viable cells was counted using a hemocytometer. As a result, it was found that treatment of cells with mPTP inhibitors reduces at least 90% of cell death seen with BPTES treatment to around 20% (Figure 4C).

Next, the pH of the medium of BPTES-treated cells was made weakly basic (pH 8.0 or pH 8.5), and the cell viability was examined.

Senescent cells and normal cells were separately seeded in 6-cm culture dishes such that approximately 10,000 cells were in each dish. On the following day, the medium was replaced with a medium containing BPTES (final concentration: 10 µM) and having pH 7.4, pH, 8.0, or pH 8.5, and culture was performed at 37°C and 5% CO₂ for 24 hours. Thereafter, the cells were stained with trypan blue every 24 hours, and the number of viable cells was counted using a hemocytometer. As a result, it was found that at least 90% of cell death seen with BPTES treatment is reduced to about 30% under weakly basic pH conditions (Figure 4D).

In addition, suppression of BNIP3 expression using the RNAi method also confirmed selective cell death and suppression of SASP in similar senescent cells.

From the above results, it was considered that glutaminolysis controls the homeostasis of pH of senescent cells through the production of ammonia, thereby maintaining the survival of the cells.

### 2-4. Removal of senescent cells by inhibiting glutaminolysis

It was examined whether BPTES treatment could remove senescent cells *in vivo.*

BPTES (12.5 mg / kg body weight) was administered to 96-week-old C57BL6/N male mice once a week for 1 month. Then, each mouse was dissected, RNA was extracted by homogenizing the heart, brain, kidney, and liver, and total RNA was prepared using the RNeasy mini kit (Qiagen) according to the attached protocol and reverse transcribed into cDNA using the ReverTra Ace qPCR RT kit (Takara). Using the obtained cDNA, the expression level of p16 mRNA, which is a marker of senescent cells, was analyzed by qPCR using Power SYBR Green PCR Master Mix (Applied Biosystems). The p16 mRNA expression level was corrected by the value of GAPDH. Primers for detecting the p16 mRNA expression level are shown below.
Forward: 5'-CCGCTGCAGACAGACTGG-3' (SEQ ID NO: 7)
Reverse: 5'-CCATCATCATCACCTGAATCG-3' (SEQ ID NO: 8)

All mice were maintained in a specific pathogen-free environment and treated according to the animal experiment guidelines of the Institute of Medical Science, The University of Tokyo (the same applies to the experiments in 2-5).

As a result of the analysis, the p16 mRNA expression was decreased by BPTES treatment in the heart, brain, kidney, and liver (Figure 5). The results indicate that the glutaminase inhibitor can remove *in vivo* senescent cells.

### 2-5. Glutaminolysis inhibitory effects on aging or age-associated organ dysfunction

It is examined whether removal of senescent cells by inhibition of glutaminolysis could improve aging or age-associated organ dysfunction. C57BL/6N male mice (8 weeks old (young mice), 76 weeks old (aged mice)) were intraperitoneally administered with BPTES (0.25 mg / 20 g / 200 µl) or a vehicle (10% DMSO (in corn oil)/200 µl) 2 or 3 times for 1 month, and their organs and blood were collected.

### 2-5-1. Effects of glutaminolysis inhibitors on age-associated dysfunction of kidney, lung, heart, and liver

The kidney, lung, liver, and heart were embedded with an OCT compound, thereby preparing frozen sections. Thereafter, tissue staining with hematoxylin-eosin (H&E), tissue staining with a Masson trichrome (MT) reagent, a periodic acid Schiff (PAS) reagent (Fisher Scientific), or immunostaining with DAB (3,3'-diaminobenzidine tetrahydrochloride) (DAKO) using an anti-F4-80 antibody (CST) was performed. After staining, tissue sections were observed under a microscope.

Kidney glomerulosclerosis was evaluated for 40 glomeruli per individual based on PAS-positive intensity and range. In addition, the serum urea concentration and creatinine concentration were measured using the Urea Assay kit (Abcam) and the Creatinine Assay kit (Abcam), respectively.

Figure 6A shows the PAS staining results of glomeruli of young mice (Young), vehicles (Mock), or aged mice (Aged) treated with BPTES (BPTES). The glomeruli of control mice were more hardened than the glomeruli of young mice, but the degree of glomerulosclerosis was improved in BPTES-treated mice (Figure 6B). It was also found that the serum urea and creatinine concentrations were also reduced by BPTES administration (Figures 6C and 6D).

The degree of lung fibrosis was assessed by MT staining. Figure 7A shows the results of MT staining of lung tissue of young mice (Young), vehicle-treated aged mice, and BPTES-treated aged mice (Aged). In addition, when the MT-stained area was quantified by a BZ-X analyzer (Keyence), the degree of fibrosis was improved in the lungs of BPTES-treated mice as compared with the lungs of control mice (Figure 7B, top).

For the heart, MT staining of heart tissue sections was performed so as to assess the degree of fibrosis (Figure 8A). In addition, the heart weight was weighed, and the cardiomyocyte size was measured with a BZ-X analyzer (Keyence). As for the heart, the degree of fibrosis in the heart of BPTES-treated mice was improved as compared with the control mice (Figure 8B). The cardiomyocyte size of BPTES-treated mice was smaller than that of control mice, and the heart weight of BPTES-treated mice was lighter than that of young mice (Figures 8C and 8D).

The effects of BPTES on macrophage infiltration into the liver with aging was investigated. Liver tissue sections were immunostained with an anti-F4/80 antibody (Figure 9A) so as to assess the degree of macrophage infiltration. The degree of macrophage infiltration in the liver of BPTES-treated mice was improved compared to control mice (Figure 9B).

The above results revealed that age-associated dysfunction of the kidney, lung, heart, and liver are ameliorated by glutaminolysis inhibitors.

### 2-5-2. Effects of glutaminolysis inhibitors on age-associated accumulation of senescent cells in white adipose tissue

Senescent cells present in white adipocyte tissue were stained with SA-β-gal (senescence-associated beta-galactosidase), and the percentage of stained cells was calculated.

In situ staining of white adipose tissue was performed as follows. Small pieces of adipose tissue were collected in PBS, fixed with 2% formamide/0.2% glutaraldehyde for 15 minutes, washed, and incubated in a newly prepared SA-β-gal stain solution (1 mg X-gal/ml, 40 mM citric acid/sodium phosphate (pH 6.0), 5 mM potassium ferrocyanide, 5 mM potassium ferricyanide, 150 mMNaCl, 2 mM MgCl₂) at 37°C for 12 hours. Then, the tissue pieces were washed with PBS and pressed between slide glasses for microscopic observation.

The abundance ratio of SA-β-gal positive cells was calculated as the ratio of the number of nuclei of positive cells to the number of nuclei of total cells using the nucleus as an indicator.

Figure 10A shows the results of SA-β-gal staining of white adipose tissue, and Figure 10B shows the proportion of SA-β-gal positive cells. The results showed that the accumulation of senescent cells stained with SA-β-gal was improved by glutaminolysis inhibitors.

### 2-5-3. Effects of glutaminolysis inhibitor on obesity-associated accumulation of senescent cells in white adipose tissue, macrophage infiltration, and white adipose tissue hypertrophy

Eight-week-old male mice (C57BL/6N) were maintained on a high-fat diet (HFD32, CLEA Japan) or a normal diet for 8 weeks. In the latter 4 weeks of the 8-week maintaining period, BPTES (0.25 mg / 20 g /200 µl) or a vehicle (10% DMSO (in corn oil)/200 µl) was intraperitoneally administered 3 times a week. Then, white adipose tissue was collected from mice, and the accumulation of senescent cells was examined by SA-β-gal staining, and macrophage infiltration was examined by immunostaining with the anti-F4/80 antibody. *In situ* staining with SA-β-gal and the determination of the abundance ratio of SA-β-gal positive cells were carried out as described in 2-5-2. The degree of macrophage infiltration was determined as described in 2-5-1. The adipocyte size was measured with a BZ-X analyzer (Keyence).

Administration of BPTES reduced the accumulation of senescent cells associated with obesity (Figures 11A and 11B) and the degree of macrophage infiltration (Figures 11A and 11E). In addition, the size of adipocytes associated with obesity was reduced (Figure 11D), and the weight of white adipose tissue was also reduced (Figure 11E).

The above results showed that glutaminolysis inhibitors improve obesity-associated senescent cell accumulation in white adipose tissue, macrophage infiltration, and white adipose tissue hypertrophy.

### 2-5-4. Effects of glutaminolysis inhibitor on obesity-associated atherosclerosis

C57BL/6J ApoE knockout mice (8 weeks old) were maintained on an atherogenetic diet (D12108C, Research Diets Inc.) for 8 weeks. In the latter 4 weeks of the 8-week maintaining period, BPTES (0.25 mg / 20 g / 200 µl) or a vehicle (10% DMSO (in corn oil)/200 µl) was intraperitoneally administered 3 times a week. In addition, C57BL/ 6J wild-type male mice (8 weeks old) were maintained on a normal diet as controls. All aortas except the arterial arch were cleanly depleted of adventitial fat, incised, and fixed flat in 4% paraformamide at 25°C for 12 hours. The aorta was washed with 70% ethanol for 5 minutes, incubated in 0.5% Sudan IV (in 1:1 acetone/ethanol) for 5 minutes, and then washed 3 times with 80% ethanol for 1 minute. Plaque formed in the aorta was stained with Sudan IV, the Sudan IV-positive area was quantified with ImageJ, and the plaque number was counted under a microscope.

As a result, it was confirmed that administration of BPTES reduces the plaque area and plaque number (Figures 12A, 12B, and 12C).

The above results showed that glutaminolysis inhibitors improve obesity-associated atherosclerosis.

### 2-5-5. Effects of glutaminolysis inhibitor on liver dysfunction associated with non-alcoholic steatohepatitis

Eight-week-old male mice (C57BL/6N) were maintained on a choline-deficient L-amino acid-defined high-fat diet (A06071302, Research Diets Inc.) for 8 weeks. In the latter 4 weeks of the 8-week maintaining period, BPTES (0.25 mg / 20 g / 200 µl) or a vehicle (10% DMSO (in corn oil)/200 µl) was intraperitoneally administered 3 times a week. The serum AST level and the hydroxyproline (OH-Pro) level in the liver were measured by the AST assay kit (Abcam) and the Hydroxyproline assay kit (Abcam), respectively. In addition, the expression levels of p16, KGA, and IL-6 were measured by qPCR. Primers for qPCR are shown below.

### p16

Forward: 5'-CGCAGGTTCTTGGTCACTGT-3' (SEQ ID NO: 9)
Reverse: 5'-TGTTCACGAAAGCCAGAGCG-3' (SEQ ID NO: 10)

### KAG

Forward: 5'-ACTGGAGATGTGTCTGCCCTCCGAAG-3' (SEQ ID NO: 11)
Reverse: 5'-CCAAAGTGTAGTGCTTCATCCATGGGG-3' (SEQ ID NO: 12)

### IL-6

Forward: 5'-CCAAGAGGTGAGTGCTTCCC-3' (SEQ ID NO: 13)
Reverse: 5'-CTGTTGTTCAGACTCTCTCCCT-3' (SEQ ID NO: 14)

It was confirmed that BPTES administration lowers serum AST levels and hydroxyproline levels in the liver (Figures 13A and 13B), as well as p16, KGA, and IL-6 expression levels (Figures 13C, 13D, and 13E).

The above results showed that the liver dysfunction associated with non-alcoholic steatohepatitis is ameliorated by glutaminolysis inhibitors.

When the experimental results using the above mice were combined, it was suggested that the removal of senescent cells by inhibiting glutaminolysis improves various aging and age-associated organ dysfunction.

### Industrial Applicability

The present invention provides a method for efficiently preparing a purified senescent cell, an agent for removing a senescent cell, an agent for preventing or treating a disease that develops with aging, and the like. Therefore, the present invention is expected to be used in the medical field.

## Claims

1. An agent for removing a senescent cell, which is a drug for removing an *in vivo* senescent cell, the agent containing an inhibitor for glutaminase as an active ingredient.

2. The agent for removing a senescent cell according to claim 1, wherein the glutaminase is kidney-type glutaminase (KGA).

3. A pharmaceutical composition for preventing or treating a disease that develops with aging, which contains the agent for removing a senescent cell according to claim 1 or 2.

4. The pharmaceutical composition according to claim 3, wherein the disease is atherosclerosis, osteoporosis, cataract, glaucoma, dementia, Parkinson's disease, lung fibrosis, chronic obstructive pulmonary disease, cancer, type 2 diabetes, chronic renal failure, cardiomegaly, liver cirrhosis, sarcopenia, or emaciation.

5. A method for preparing a senescent cell, comprising the following steps (a) to (c):
(a) synchronizing a cell with the G2 phase;
(b) activating an intracellular p53 protein in the cell synchronized with the G2 phase; and
(c) inhibiting polo-like kinase 1 (PLK1) activity in the cell treated in the step (b).

6. The method according to claim 5, wherein the step (a) is a step of bringing a cyclin-dependent kinase 1 (CDK1) activity inhibitor into contact with the cell.

7. The method according to claim 5, wherein the step (b) is a step of bringing an Mdm2 protein inhibitor into contact with the cell.

8. The method according to claim 5, wherein the step (c) is a step of bringing a PLK1 activity inhibitor into contact with the cell.
